# EUROPEAN PATENT APPLICATION

(11) **EP 4 144 836 A1**
(43) Date of publication of application: **08.03.2023**
(21) Application number: 21795735.6
(22) Date of filing: 28.04.2021
(51) Int. Cl.: C12N 5/0775, C12M 1/32, C12M 3/06, C12M 1/00, A61K 35/28, A61P 29/00, A61P 3/10

(54) **METHOD FOR PRODUCING EXTRACELLULAR VESICLES FROM THREE-DIMENSIONALLY CULTURED STEM CELLS**

(30) Priority: 28.04.2020 KR 20200051640
(71) Applicant: Konkuk University Industrial Cooperation Corp., Gwangjin-gu Seoul 05029 (KR)
(72) Inventor: CHO, Ssang-Goo, Seoul 05039 (KR); KIM, Sehee, Seoul 04726 (KR); LIM, Kyung Min, Seoul 05010 (KR)
(74) Representative: V.O.
(86) International application number: PCT/KR2021/005408
(87) International publication number: WO 2021/221471

(57) **Abstract**

The present disclosure relates to a method for producing extracellular vesicles from three-dimensionally cultured stem cells. The method of the present disclosure can produce stem cell-derived extracellular vesicles with a high yield through orbital shaking culture of stem cell aggregates in the presence of TGF-β and thus can be usefully used in an industrial-scale mass production process of exosomes that can be utilized as a pharmaceutical ingredient substituting for a cell therapeutic agent. Furthermore, the exosomes obtained by the method of the present disclosure have significantly improved immunoregulatory functions as compared to the exosomes produced by the existing method and, therefore, can be applied as a superior therapeutic composition for various inflammations or autoimmune diseases.

## Description

### [Technical Field]

The present disclosure relates to a method for obtaining stem cell-derived extracellular vesicles with high yield through three-dimensional culturing of stem cells.

### [Background Art]

Extracellular vesicles are lipid bilayer-structured vesicles of various sizes released from various eukaryotic cells including human, animals, insects, plants and microorganisms. Among them, nanovesicles having nanometer-scale diameters are called exosomes. The exosomes contain specific molecules such as proteins, nucleic acids, lipids, carbohydrates, etc. of cells. They stably protect these molecules with lipid bilayers and serve to deliver signals to other cells after being released.

In regenerative medicine or immunotherapy using stem cells, a therapeutic method using stem cell-derived extracellular vesicles instead of directly transplanting living stem cells to the affected site has shown efficacy in preclinical trials and has entered the clinical phase for some diseases. It is known that the exosomes released by stem cells contain the key factors associated with anti-inflammatory activity and self-renewal activity possessed by the stem cells. Accordingly, it draws attentions as a new approach capable of overcoming the disadvantage of the existing cell therapeutic agents such as the difficulty in acquiring and maintaining a therapeutically effective amount of cells. However, the number of exosomes secreted by nucleated cells is only about 1000 per cell, in general. Accordingly, it is a very important issue to improve the yield of exosomes isolated from cells in the exosome-based therapy.

Generally, exosomes are obtained by isolating from a cell culture. In general, stem cells are cultured two-dimensionally. But, in this case, a large amount of cells should be cultured to acquire a large quantity of exosomes, which leads to increased cost. In addition, the isolation of exosomes from a large volume of cell culture is highly labor-intensive. For isolation and purification of exosomes, centrifugation and tangential flow filtration (TFF) are mainly used. Centrifugation is not suitable for isolation of exosomes from a large volume of cell culture because of limited capacity. TFF is advantageous over centrifugation in that it is suitable for a large-scale process, but there are many problems to be solved such as shear stress and loss of exosomes during the filtration. In order to solve these problems, an effective extraction method capable of obtaining a large quantity of exosome from a small number of cells or a small volume of cell culture needs to be developed.

Throughout the present specification, a number of publications and patent documents are referred to and cited. The disclosure of the cited publications and patent documents is incorporated herein by reference in its entirety to more clearly describe the state of the related art and the present disclosure.

### [Disclosure]

### [Technical Problem]

The inventors of the present disclosure have made consistent efforts to develop a method for effectively obtaining stem cell-derived extracellular vesicles, specifically mesenchymal stem cell-derived exosomes, which contain various useful ingredients and can function as stable drug delivery systems due to lipid bilayers. As a result, they have found out that, when stem cell aggregates are cultured three-dimensionally while providing a physical, biological and special environment similar to that of the *in-vivo* condition and adding TGF-β (transforming growth factor beta) superfamily cytokines, not only the yield of exosomes but also the inherent immunoregulatory effect of stem cells such as therapeutic effect for inflammatory diseases is improved greatly, and have completed the present disclosure.

The present disclosure is directed to providing a method for producing stem cell-derived extracellular vesicles.

The present disclosure is also directed to providing a composition for preventing or treating an inflammatory or autoimmune disease, which contains stem cell-derived extracellular vesicles produced by the method described above as active ingredients.

Other purposes and advantages of the present disclosure will become more apparent by the following detailed description, claims and drawings.

### [Technical Solution]

In an aspect, the present disclosure provides a method for producing stem cell-derived extracellular vesicles, which includes:
(a) a step of forming cell aggregates by culturing stem cells isolated from a subject; and
(b) a step of three-dimensionally culturing the cell aggregates in a culture medium containing TGF-β (transforming growth factor beta).

The inventors of the present disclosure have made consistent efforts to develop a method for effectively obtaining stem cell-derived extracellular vesicles, specifically mesenchymal stem cell-derived exosomes, which contain various useful ingredients and can function as stable drug delivery systems due to lipid bilayers. As a result, they have found out that, when stem cell aggregates are cultured three-dimensionally while providing a physical, biological and special environment similar to that of the *in-vivo* condition and adding TGF-β (transforming growth factor beta) superfamily cytokines, not only the yield of exosomes but also the inherent immunoregulatory effect of stem cells such as therapeutic effect for inflammatory diseases is improved greatly, and have completed the present disclosure.

In the present specification, the term "extracellular vesicles" refers to lipid bilayer-structured vesicles with a diameter of 30-1,000 nm, which are released from various eukaryotic cells to the extracellular environment through fusion of the multivesicular body with the plasma membrane.

In a specific exemplary embodiment of the present disclosure, the extracellular vesicles produced by the method of the present disclosure have an average diameter of 30-150 nm, more specifically 50-120 nm. The extracellular vesicles having a diameter of the above-described ranges are called exosomes.

In the present specification, the term "stem cells" collectively refer to undifferentiated cells prior to differentiation into individual cells constituting tissues, which have the ability to differentiate into specific cells under specific stimulation (environment). Unlike differentiated cells whose cell division has stopped, the stem cells retain the capability of self-renewal through cell division and have the plasticity of differentiation to differentiate into various cells under different stimuli.

The stem cells used in the present disclosure may be any cells that have the characteristics of stem cells, i.e., being undifferentiated, unlimited proliferation and ability to differentiate into specific cells, and thus can be induced to differentiate into the tissue desired to be regenerated, without limitation.

In a specific exemplary embodiment of the present disclosure, the stem cells used in the present disclosure are mesenchymal stem cells.

In the present specification, the term "mesenchymal stem cells" refer to stem cells having the multipotency of differentiating into fat cells, bone cells, cartilage cells, muscle cells, nerve cells and cardiac muscle cells. The mesenchymal stem cells can be distinguished by their spiral shapes and the expression level of the basic cell surface markers CD73(+), CD105(+), CD34(-) and CD45(-). They have the function of regulating immune response in addition to the multipotency.

In a specific exemplary embodiment of the present disclosure, the step (a) is performed by suspension-culturing stem cells in a multi-well culture plate.

In the present specification, the term "suspension culture" refers to culturing of cells in a floating state in a culture medium without adhesion to a substrate, etc. Thus, the term "suspension culture" is used in the same meaning as "three-dimensional culture". Adhesion-dependent stem cells tend to aggregate during suspension culture. Because the cells that float without participating in the aggregation die of apoptosis, an environment corresponding to the adhesion property should be created. According to the present disclosure, by suspension-culturing stem cells in a multi-well plate having a plurality of wells, cell aggregates of different sizes can be formed in the wells of different sizes. Therefore, standardized stem cell aggregates having identical size and shape can be obtained in large quantities.

In the present specification, the term "cell aggregate" refers to a cell mass of a three-dimensional structure formed as cells cultured in an environment allowing three-dimensional growth, such as suspension culture, etc., rather than monolayer growth are self-aggregated. The cell aggregate obtained through three-dimensional culturing provides an environment similar to that of the tissue from which the stem cells are derived. Depending on the size and the number of the self-aggregated cells, it may have a spherical or other shape. The cell aggregate having a spherical shape is called a spheroid. But, the spheroid needs not to be perfectly spherical geometrically.

In the present specification, the term "cell culture" refers to a mixture for growth and proliferation of cells *in vitro,* which contains ingredients essential for the growth and proliferation of cells such as sugars, amino acids, various nutrients, minerals, etc.

The ingredients that can be further contained in a cell culture medium include, for example, glycerin, L-alanine, L-arginine hydrochloride, L-cysteine hydrochloride monohydrate, L-glutamine, L-histidine hydrochloride monohydrate, L-lysine hydrochloride, L-methionine, L-proline, L-serine, L-threonine, L-valine, L-asparagine monohydrate, L-aspartic acid, L-cystine 2HCl, L-glutamic acid, L-isoleucine, L-leucine, L-phenylalanine, L-tryptophan, L-tyrosine disodium salt dihydrate, i-inositol, thiamine hydrochloride, niacinamide, pyridoxine hydrochloride, biotin, calcium D-panthothenate, folic acid, riboflavin, vitamin B₁₂, sodium chloride (NaCl), sodium bicarbonate (NaHCOs), potassium chloride (KCI), calcium chloride (CaCl₂), sodium dihydrogen phosphate monohydrate (NaH₂PO₄·H₂O), copper sulfate pentahydrate (CuSO₄·5H₂O), iron(II) sulfate heptahydrate (FeSO₄·7H₂O), magnesium chloride (anhydrous), magnesium sulfate (MgSO₄), disodium hydrogen phosphate (Na₂HPO₄), zinc sulfate heptahydrate (ZnSO₄·7H₂O), D-glucose (dextrose), sodium pyruvate, hypoxanthine Na, linolenic acid, lipoic acid, putrescine 2HCl and thymidine, although not being limited thereto.

The cell culture medium according to the present disclosure may be prepared artificially or may be purchased from commercially available ones. Examples of the commercially available culture medium include IMDM (Iscove's modified Dulbecco's medium), α-MEM (alpha modification of Eagle's medium), F12 (nutrient mixture F-12) and DMEM/F12 (Dulbecco's modified Eagle's medium: nutrient mixture F-12), although not being limited thereto.

In a specific exemplary embodiment of the present disclosure, each well of the multi-well culture plate has a size of 300-500 µm, more specifically 350-450 µm, most specifically about 400 µm.

In a specific exemplary embodiment of the present disclosure, the suspension culture is performed by seeding 300⁻500 cells, more specifically 350-450 cells, most specifically about 400 cells, per each well of the multi-well culture plate.

In a specific exemplary embodiment of the present disclosure, the TGF-β used in the present disclosure is TGF-β1, TGF-β2 or TGF-β3, more specifically TGF-β3.

In a specific exemplary embodiment of the present disclosure, the step (b) is performed by orbital shaking-culturing the cell aggregate in floating state.

More specifically, the orbital shaking culture is performed at 50-70 rpm, further more specifically at 53-67 rpm, even more specifically at 55-65 rpm, most specifically at 57-63 rpm.

In a specific exemplary embodiment of the present disclosure, the method of the present disclosure further includes a step of isolating the extracellular vesicles from the culture medium obtained in the step (b) through multiple times of centrifugation.

Whereas it is difficult to obtain a sufficient amount of extracellular vesicles through centrifugation from stem cells cultured according to the existing method due to the limitation of capacity, a therapeutically effective amount of extracellular vesicles can be obtained easily only through centrifugation because the amount of extracellular vesicles released from the stem cells cultured according to the method of the present disclosure is increased remarkably.

In another aspect, the present disclosure provides stem cell-derived extracellular vesicles produced by the method of the present disclosure described above.

In another aspect, the present disclosure provides a composition for preventing or treating an inflammatory or autoimmune disease, which contains the stem cell-derived extracellular vesicles produced by the method of the present disclosure described above as active ingredients.

A detailed description of the stem cell-derived extracellular vesicles used in the present disclosure will be omitted to avoid unnecessary redundancy.

In the present specification, the term "prevention" refers to suppressing the onset of a disorder or a disease in a subject who has not been diagnosed with the disorder or disease but is susceptible to the disorder or disease.

In the present specification, the term "treatment" refers to (a) suppressing the development of a disorder, a disease or symptoms; (b) reducing the disorder, disease or symptoms; or (c) removing the disorder, disease or symptoms. The composition of the present disclosure suppresses the development of the symptoms of various inflammatory or autoimmune diseases caused by excessive or unwanted immune response by effectively suppressing T-cell mediated immune activity, or removes or reduces them. Accordingly, the composition of the present disclosure may be used as a composition for treating a disease on its own, or may be used as a therapeutic adjuvant for the disease when administered with in combination with another pharmaceutical ingredient having therapeutic effect for the inflammatory or autoimmune disease. Therefore, in the present specification, the term "treatment" or "therapeutic agent" encompasses "assistance of treatment" or "therapeutic adjuvant".

In the present specification, the term "administration" refers to direct administration of a therapeutically effective amount of the composition of the present disclosure to a subject, so that the same amount is formed in the body of the subject. It has the same meaning as "transplantation" or "injection".

In the present specification, the term "therapeutically effective amount" refers to the amount of the composition of the present disclosure which is sufficient to provide a therapeutic or prophylactic effect in a subject. Therefore, the term encompasses "prophylactically effective amount".

In the present specification, the term "subject" includes human, mouse, rat, guinea pig, dog, cat, horse, cow, pig, monkey, chimpanzee, baboon or rhesus monkey without limitation. Specifically, the subject of the present disclosure is human.

In a specific exemplary embodiment of the present disclosure, the inflammatory or autoimmune disease prevented or treated by the composition of the present disclosure includes, for example, rheumatoid arthritis, reactive arthritis, type 1 diabetes, type 2 diabetes, systemic lupus erythematosus, multiple sclerosis, cryptogenic fibrosing alveolitis, polymyositis, dermatomyositis, localized scleroderma, systemic scleroderma, colitis, inflammatory bowel disease, Sjorgen's syndrome, Raynaud's phenomenon, Bechet's disease, Kawasaki's disease, primary biliary sclerosis, primary sclerosing cholangitis, ulcerative colitis, graft-versus-host disease (GVHD) and Crohn's disease, although not being limited thereto.

When the composition of the present disclosure is prepared as a pharmaceutical composition, the pharmaceutical composition of the present disclosure contains a pharmaceutically acceptable carrier.

The pharmaceutical composition of the present disclosure contains a pharmaceutically acceptable carrier which is commonly used in preparation of a pharmaceutical composition, e.g., lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, etc., although not being limited thereto. The pharmaceutical composition of the present disclosure may further contain a lubricant, a wetting agent, a sweetener, a flavorant, an emulsifier, a suspending agent, a preservative, etc. in addition to the above-described ingredients. Suitable pharmaceutically acceptable carriers and preparations are described in detail in Remington's Pharmaceutical Sciences (19th ed., 1995).

The pharmaceutical composition of the present disclosure may be administered orally or parenterally. Specifically, it may be administered orally, intravenously, subcutaneously or intraperitoneally.

An appropriate administration dosage of the pharmaceutical composition of the present disclosure will be prescribed in consideration of various factors such as formulation method, administration method, the age, body weight, sex, pathological condition and diet of a patient, administration time, administration route, excretion rate and response sensitivity. A preferred administration dosage of the pharmaceutical composition of the present disclosure for an adult is within a range of 0.001-100 mg/kg.

The pharmaceutical composition of the present disclosure may be prepared according to a method that can be easily carried out by those having ordinary knowledge in the art to which the present disclosure belongs into a single-dose or multiple-dose formulation using a pharmaceutically acceptable carrier and/or excipient. The formulation may be a solution in an oily or aqueous medium, a suspension, a syrup, an emulsion, an extract, a dust, a powder, a granule, a tablet or a capsule, and may further contain a dispersant or a stabilizer.

In another aspect, the present disclosure provides a method for preventing or treating an inflammatory or autoimmune disease, which includes a step of administering a composition containing the stem cell-derived extracellular vesicles of the present disclosure as active ingredients to a subject.

In another aspect, the present disclosure provides stem cell-derived extracellular vesicles highly expressing one or more protein selected from a group consisting of peroxiredoxin-4, thioredoxin reductase 1 and prostaglandin G/H synthase 2.

The extracellular vesicles, e.g., exosomes, isolated from the mesenchymal stem cells cultured by the method of the present disclosure, show distinct difference in protein expression profiles from the exosomes obtained by the existing method. As shown in the examples described below, the three proteins mentioned above are highly expressed significantly as compared to in mesenchymal stem cell-derived exosomes obtained by three-dimensional culturing only without two-dimensional culture or TGF-β3 treatment. That is to say, the exosomes obtained by the method of the present disclosure have a novel composition as compared to the exosomes obtained by the existing method.

In the present specification, the term "highly expressing" means that the content, secretin level or expression level of specific proteins in the exosomes obtained by the method of the present disclosure is increased significantly as compared to in the mesenchymal stem cell-derived exosomes obtained by the existing method. Specifically, it means that the content, secretin level or expression level has increased by 40% or more, more specifically by 60% or more, further more specifically by 80% or more, most specifically by 100% or more.

In a specific exemplary embodiment of the present disclosure, the extracellular vesicles further highly express one or more protein selected from a group consisting of heat shock protein 90-β (HSP90-β), neprilysin, T-complex protein 1 (TCP1) subunit α, filamin A, 40S ribosomal protein S3, myosin-9, transaldolase, fascin, thioredoxin reductase 1 and RuvB-like 2 (RUVBL 2).

In a specific exemplary embodiment of the present disclosure, the extracellular vesicles are positive for one or more protein selected from a group consisting of coronin 1A, prolyl 4-hydroxylase subunit α-2 and purine nucleoside phosphorylase. According to the present disclosure, the three proteins mentioned above are not detected in mesenchymal stem cell-derived exosomes obtained by three-dimensional culturing only without two-dimensional culture or TGF-β3 treatment. That is to say, the exosomes obtained by the method of the present disclosure have an entirely different new protein expression profile.

### [Advantageous Effects]

The features and advantages of the present disclosure may be summarized as follows:
(a) The present disclosure provides a method for producing extracellular vesicles from three-dimensionally cultured stem cells.
(b) Since the method of the present disclosure allows the obtainment of stem cell-derived extracellular vesicles with high yield through orbital shaking culture of stem cell aggregates in the presence of TGF-β, it can be usefully used in an industrial-scale mass production process of exosomes that can be utilized as a pharmaceutical ingredient substituting for a cell therapeutic agent.
(c) Furthermore, the exosomes obtained by the method of the present disclosure have significantly improved immunoregulatory functions as compared to the exosomes produced by the existing method and, therefore, can be applied as a superior therapeutic composition for various inflammations or autoimmune diseases.

### [Brief Description of Drawings]

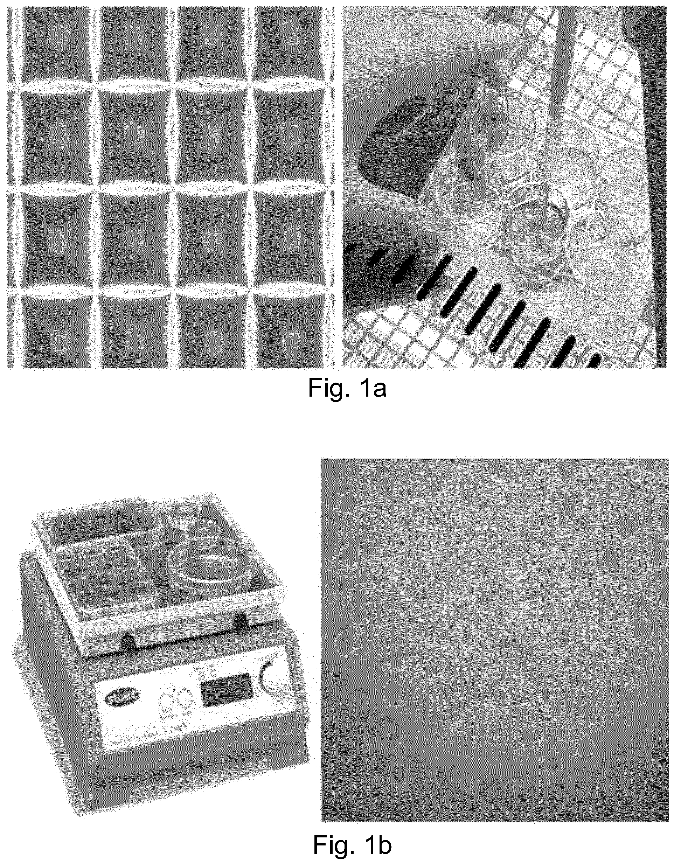

FIGS. 1a and 1b show three-dimensional culturing of mesenchymal stem cells according to a method of the present disclosure. They show the formation of embryonic bodies (FIG. 1a) and 3D culturing using an orbital shaker (FIG. 1b).
FIG. 2 shows the yield of exosomes under different culture conditions.
FIG. 3 shows the change of PDI values depending on the treatment with TGF-β. It can be seen that one peak appears under a 3D shaking culture condition with TGF-β treatment.

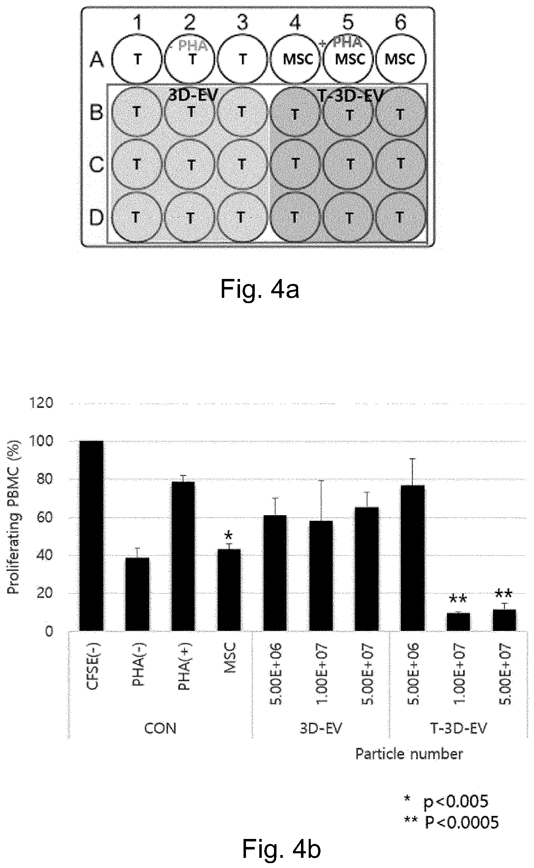

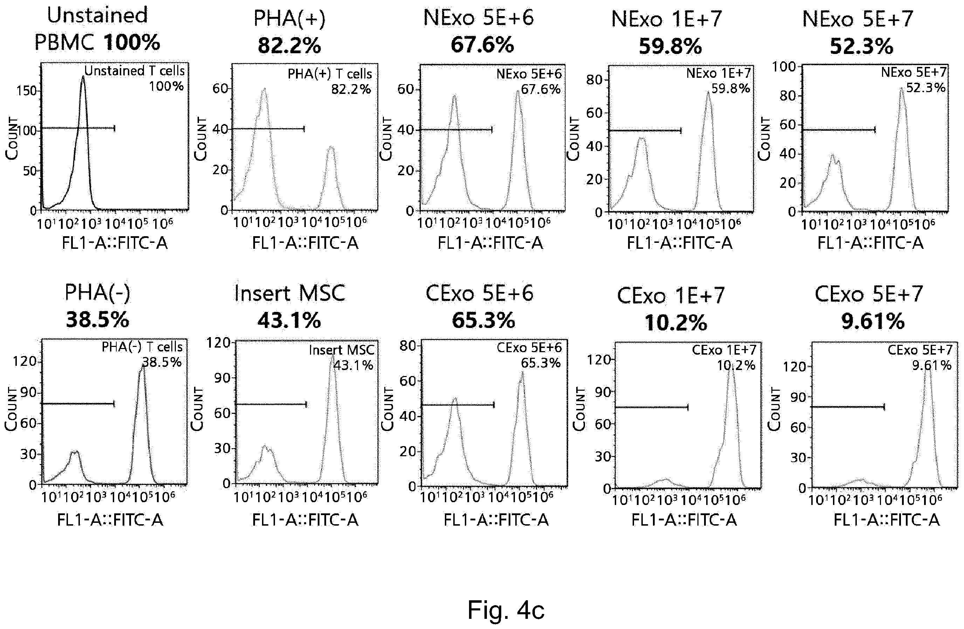

FIGS. 4a-4c show the effect of TGF-β on proliferation of T cells. After inducing the proliferation of PBMCs using PHA, T cell-inhibiting effect was investigated for the culture media of a negative control group (untreated group), a positive control group (MSC treatment group), exosomes obtained by 3D shaking culture only (3D-EV), and exosomes obtained by 3D shaking culture with addition of TGF-β3 (T-3D-EV) (FIG. 4a). As a result, it was confirmed that the exosomes obtained by 3D shaking culture with addition of TGF-β3 had the most noticeable T cell-inhibiting effect (FIGS. 4b and 4c). This shows that the exosomes obtained by the method of the present disclosure have improved function as well as high yield.
FIG. 5a shows a result of investigating the size of exosomes through dynamic light scattering (DLS) analysis. FIG. 5b shows a result of observing the shape and structure of exosomes by transmission electron microscopy (TEM). FIG. 5c shows a result of western blotting analysis for investigating the expression of CD9, CD63, flotillin-1 and Alix. FIG. 5d shows a result of immunophenotyping analysis for the surface of exosomes through flow cytometry.
FIGS. 6a and 6b show a result of investigating the wound-healing ability of keratinocytes (HaCaT cells) by the exosomes of the present disclosure.
FIG. 7 shows proteins changing specifically in T-3D-EV samples, which are exosomes obtained by a method of the present disclosure, among four clusters identified through clustering analysis.
FIG. 8 shows a comparative analysis result of the biological characteristics of proteins showing specific expression profiles in exosomes of the present disclosure.
FIG. 9 shows the biological functions and immune-related characteristics of proteins of three identified clusters.
FIG. 10 shows the degree of discrimination between groups identified through principal component analysis using discrimination indices.
FIG. 11 shows a result of gene set enrichment analysis, showing enriched gene sets, normalized enrichment scores (NES) and p-values.
FIG. 12 shows gene groups enriched in the present disclosure.
FIGS. 13 and 14 show proteins whose expression has increased or decreased 2 times or more in exosomes obtained by a method of the present disclosure as compared to a 3D culture group (FIG. 13) and proteins expressed only in the exosomes obtained by the method of the present disclosure (FIG. 14).
FIG. 15 shows the expression profile of inflammation-associated proteins in exosomes obtained by a method of the present disclosure.

### [Best Mode]

Hereinafter, the present disclosure will be described in more detail through examples. The examples are only for describing he present disclosure specifically, and it will be obvious to those having ordinary knowledge in the art that the scope of the present disclosure is not limited by the examples.

### Examples

### Example 1: Three-dimensional culture of mesenchymal stem cells

For 3D culturing of stem cells, AggreWell^{™}400 (STEMCELL Technologies; #34425) having about 7000 microwells with a size of 400 µm treated with an F127 solution and uniform spheroids having a diameter of 120-200 µm were produced by seeding about 400 umbilical cord-derived mesenchymal stem cells per well (Seoul National University Hospital, Konkuk University Bioethics Committee: 001355-201705-BR-181). The spheroids were seeded in a culture medium containing TGF-β3 on a non-absorbent culture dish and then shaking-cultured for 3 days at 60 rpm and 37 °C using an orbital shaker (INFORS HT Celtron; #69455). 3 days later, exosomes were isolated from the culture medium.

### Example 2: Isolation and quantification of exosomes

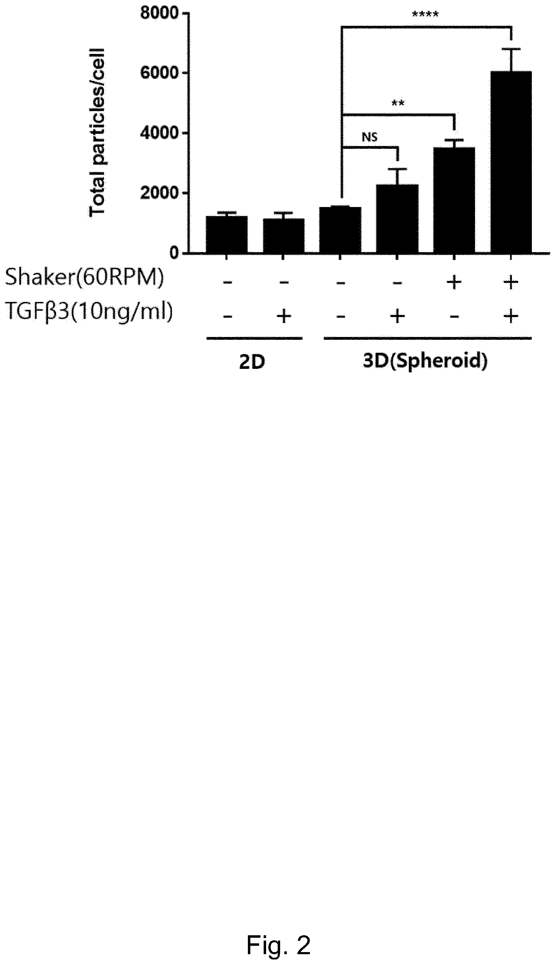

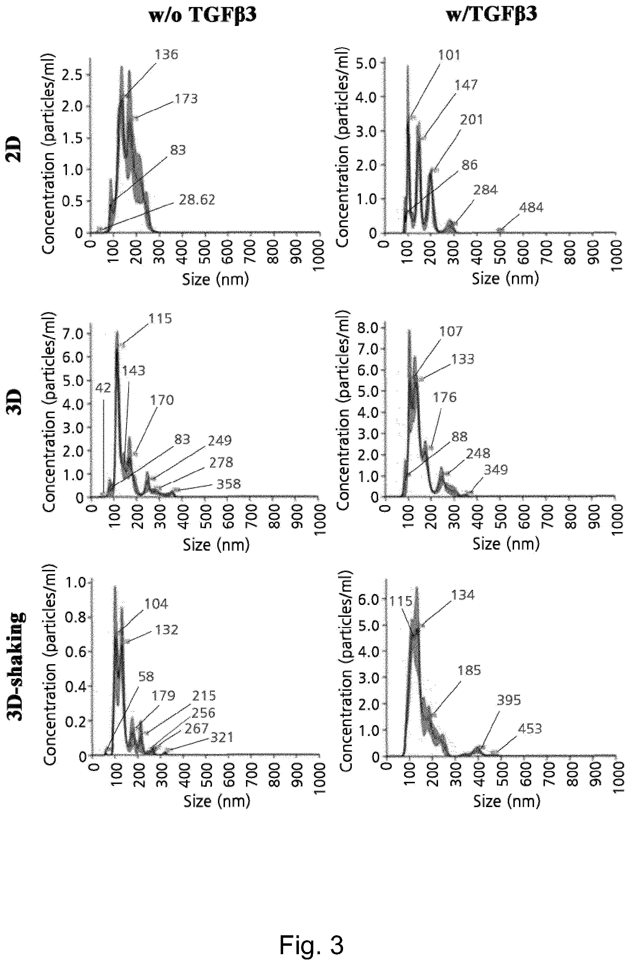

After removing cell debris by centrifuging the culture medium at 300 g for 10 minutes, followed by centrifugation at 2000 g for 10 minutes, the supernatant was transferred to a fresh tube and centrifuged again at 10,000 g for 30 minutes. After centrifuging the supernatant again at 187,000 g for 2 hours, exosomes were obtained from the resulting pellets. After isolating the exosomes from the culture medium, the number and peaks of the exosomes were investigated by conducting nanoparticle tracking analysis (NTA; NS300, NanoSight System) according to the manufacturer's instructions. As a result, when 3D culture was applied without shaking (60 rpm), the yield was not significantly different from that of general 2D culture, suggesting that the effect of the three-dimensional culturing itself is not significant. Significant increase in the yield was confirmed for the group to which all of 3D culture, shaking (60 rpm) and TGF-β3 addition were applied as compared to when only TGF-β3 was added during 3D culture without shaking (60 rpm) or when only 3D shaking culture was applied without addition of TGF-β3 (FIG. 2). In addition, one peak appeared when 3D shaking culture was performed with TGF-β treatment, suggesting that homogenous exosomes were produced (FIG. 3).

### Example 3: PBMC proliferation assay

It was investigated whether the exosomes obtained from the culture medium treated with TGF-β obtained in Example 2 have T cell-inhibiting effect as compared to a control group (normal cell culture) by conducting PBMC proliferation assay according to a previously reported method (Hsu, P.J., et al. J. Vis. Exp. (106), e53265, doi:10.3791/53265(2015)). After isolating PBMCs from blood (Konkuk University Hospital, Konkuk University Bioethics Committee: 7001355-201705-BR-181) using ficoll and culturing for 5 days, the PBMCs were stained with CFSE (carboxyfluorescein succinimidyl ester, Invitrogen; #C34554) and the proliferation of the PBMCs was investigated by flow cytometry. After inducing an inflammatory environment accompanied by T cell proliferation by treating with PHA (phytohemagglutinin, Sigma; #L1668), the PBMC-inhibiting effect of exosomes obtained from a normal cell culture (EV), the exosomes obtained by 3D shaking culture only (3D-EV) and the exosomes obtained by 3D shaking culture with addition of TGF-β3 (T-3D-EV) was investigated with respect to the PBMC-inhibiting effect of mesenchymal stem cells as a positive control group (FIG. 4a). As a result, the exosomes of the present disclosure obtained by 3D shaking culture with TGF-β treatment showed the best T cell-inhibiting effect close to 80% with respect to that of the positive control group by decreasing the proliferative T cells of the MSC treatment group from 43.1 % to 9.6% (FIGS. 4b and 4c). Therefore, it can be seen that the exosomes obtained by the method of the present disclosure have improved function as well as high yield.

### Example 4: Characterization of exosomes

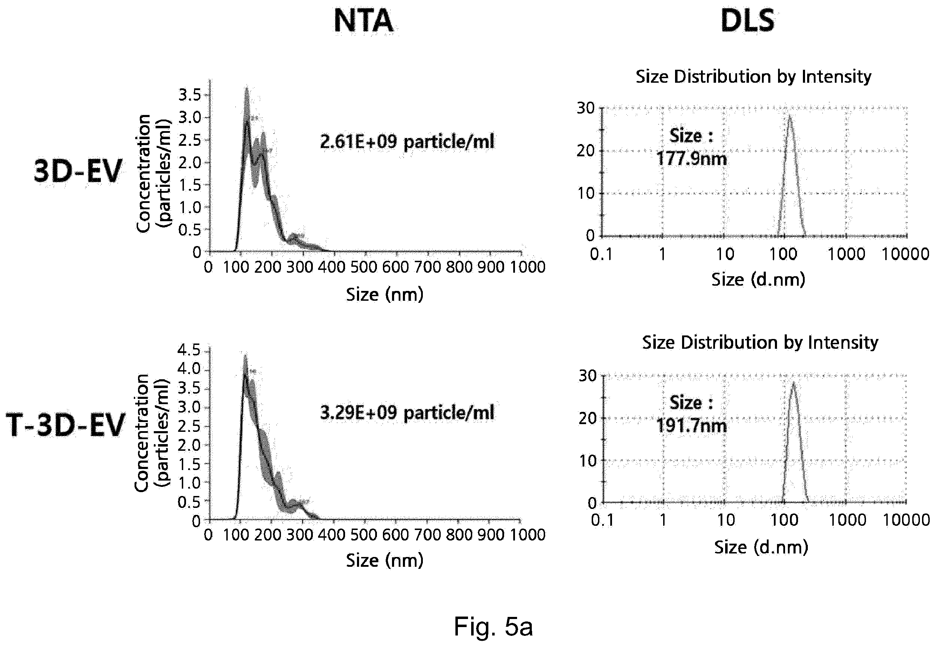

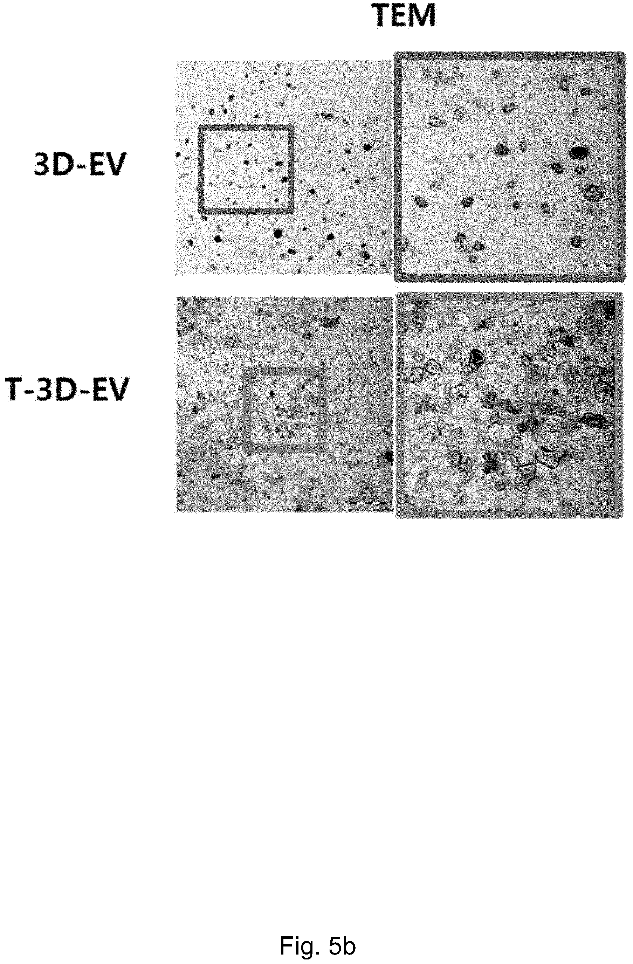

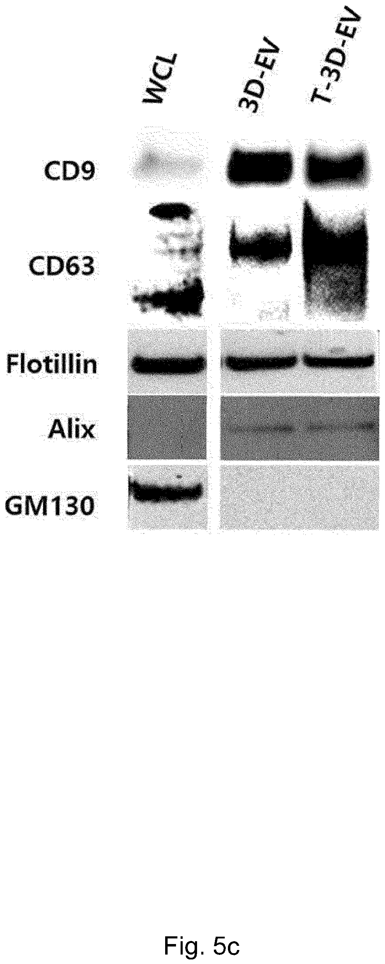

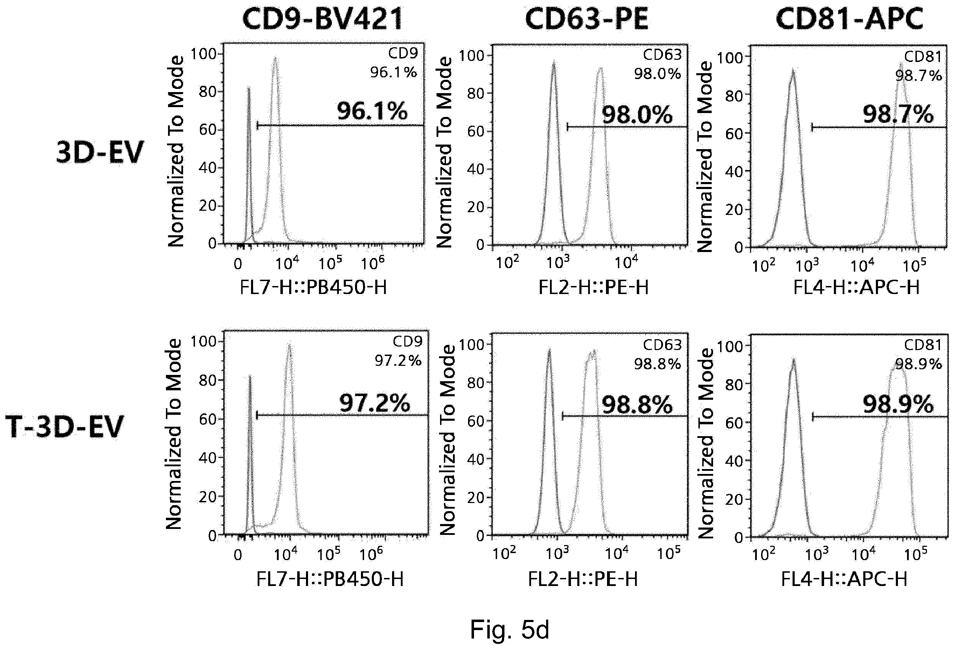

The size of the exosomes was investigated by dynamic light scattering (DLS) using Nano Zetasizer (Malvern Instruments, Malvern, UK), and the number of the EVs was counted with the nanoparticle tracking analyzer NS300 (Nanosight, Amesbery, UK). The shape and structure of the exosomes were analyzed by at transmission electron microscopy (TEM, JEM-1010, Nippon Denshi, Tokyo, Japan) 80 kV. As a result, the exosomes were cup- or sphere-shaped (FIG. 5b).

After adhering the exosomes onto a grid (formvar/carbon 300 mesh, copper, FCF300-CU 50/pk), negative staining was performed using 1% phosphotungstic acid hydrate (Sigma, P4006). For identification of exosome-associated positive markers, the expression of CD9 (ab263023, Abcam), CD63 (ab134045, Abcam), flotillin-1 (#18634, CST) and Alix (#2171, CST) proteins was investigated by immunoblotting. As a result of western blotting, the positive markers for the exosomes were expressed, whereas the expression of the exosome negative marker GM130 (#12480, CST) protein was not observed.

Immunophenotyping analysis was conducted on the surface of the exosomes by flow cytometry. First, because the size of the exosomes is not appropriate for analysis by flow cytometry, 2.7 µm Dynabeads (10620D, Invitrogen, exosome-human CD9 flow detection reagent (from cell culture)) to which CD9 antibody, a positive marker of exosomes, is conjugated were attached to the exosomes to increase the size, which were then labeled with CD9-BV421 (BD Bioscience, 743047), CD63-PE (BD Bioscience, 556020) and CD81-APC (MACS Miltenyi Biotec, M130-119-787) antibodies. Then, the intensity of fluorescence generated by the labeled antibodies were measured using a flow cytometer (Beckman Coulter, CytoFlex flow cytometry analyzer). As a result, it was confirmed that the fluorescence intensity of CD9, CD63 and CD81 was 96% or higher. From the fact that the isolated exosomes express 96-98% of exosome positive markers, it can be seen that homogenous exosomes were isolated.

### Example 5: Wound healing assay of exosomes

For evaluation of the wound healing capacity of the exosomes in keratinocytes (HaCaT cells), HaCaT cells were inoculated onto a culture dish and, after culturing to 90% confluency, a long scratch was made using a 1000 µL tip end. Then, after replacing with a cell culture medium (Dulbecco's modified Eagle's medium-high glucose, D6429, Sigma) containing the exosomes (1E+10 particles/mL), the wound site was imaged with given time intervals.

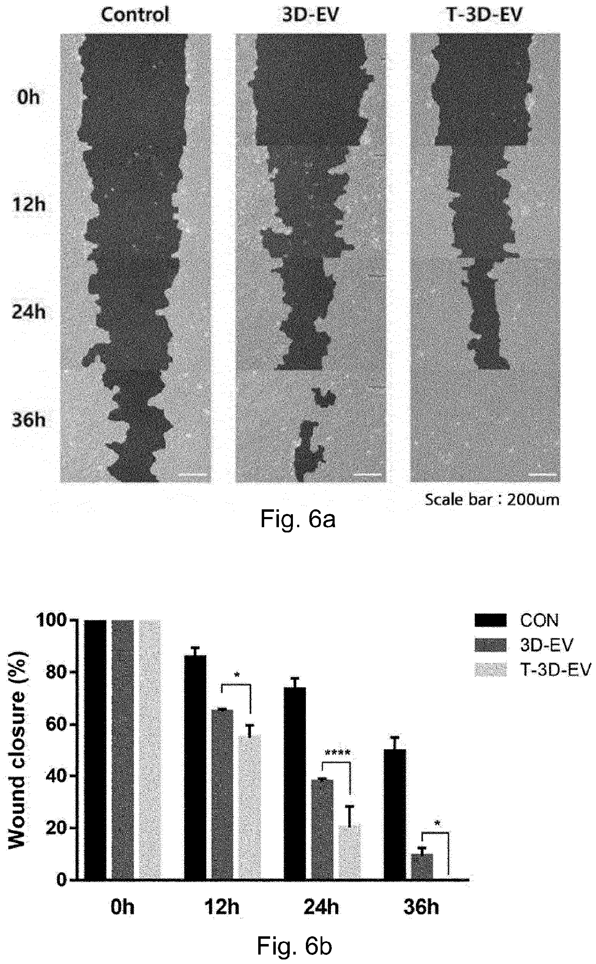

As a result, the groups treated with the exosomes showed faster wound closure than the negative control group, and the group to which TGF-β3 and 3D shaking culture were applied (T-3D-EV) showed faster wound closure than the group to which only 3D shaking culture was applied (3D-EV) (FIG. 6).

### Example 6: Analysis of proteins expressed in exosomes

### Extraction and quantitative analysis of proteins

Peptides were prepared and expressed from proteins stained in gels by the in-gel digestion method. Specifically, after destaining the proteins using a 50 mM ammonium bicarbonate/50% acetonitrile solution and a 100% acetonitrile solution, disulfide bonds were reduced at 37 °C using 50 mM dithiothreitol. Then, after conducting alkylation under light-shaded condition using 55 mM iodoacetamide, dehydration was conducted using a 100% acetonitrile solution. Then, peptides were prepared using LysS/trypsin mixed protease dissolved in 50 mM ammonium bicarbonate. The extracted peptides were dissolved in 0.1% formic acid and subjected to liquid chromatography (LC) and mass spectrometry. The mass spectrometry was performed using Q-Exactive Plus (Thermo, USA), and the liquid chromatography was performed using UltiMate^{™} 3000 RSLCnano System (Thermo, USA). The analysis was conducted by injecting 5 µL of the peptide sample to an ion trap mass spectrometer coupled with NanoLC at a flow rate of 250 µL/min. The LC was performed for a total of 200 minutes including 150 minutes of a concentration gradient of solution A (5% dimethyl sulfoxide, 0.1% formic acid) and solution B (95% acetonitrile, 0.1% formic acid, 5% dimethyl sulfoxide). A fused silica capillary column filled with C18 (2 µm, 100 Å), with an inner diameter of 75 µm, an outer diameter of 360 µm and a length of 50 cm, was used to separate the peptide mixture. The separated peptides were introduced to the mass spectrometer to obtain spectrum data. Orbitrap MS analysis was performed by repeating 20 cycles of MS/MS (resolution 17,500) using Orbitrap MS according to the HCD (higher energy collision dissociation, 27% energy level) method following one survey scan (resolution 70,000) in a range of 350-1800 m/z using an ion trap MS. The detection of overlapping peptide ions was minimized by setting the dynamic exclusion option to 20 seconds. The auto gain control target setting of the ion trap was 3E06 for full MS and 1E5 for FT MS/MS. Qualitative analysis and label-free quantitative analysis were performed for the acquired RAW file using the Andromeda algorithm-based data analysis software MaxQuant (version 1.6.10.43, https://www.maxquant.org/). For cysteine, cysteine carbamidomethylation was set as a fixed modification, while methionine oxidation was set as a variable modification. The Human SwissProt database published in October 2019 was used as a protein sequence database and the MaxLFQ algorithm was used for the label-free quantitative analysis of proteins. The identified proteome was subjected to heat mapping, clustering analysis and principal component analysis using Perseus (http://www.perseus-framework.org). The ClueGO, ShinyGO v0.60 and GSEA programs were used for characterization of the protein clusters.

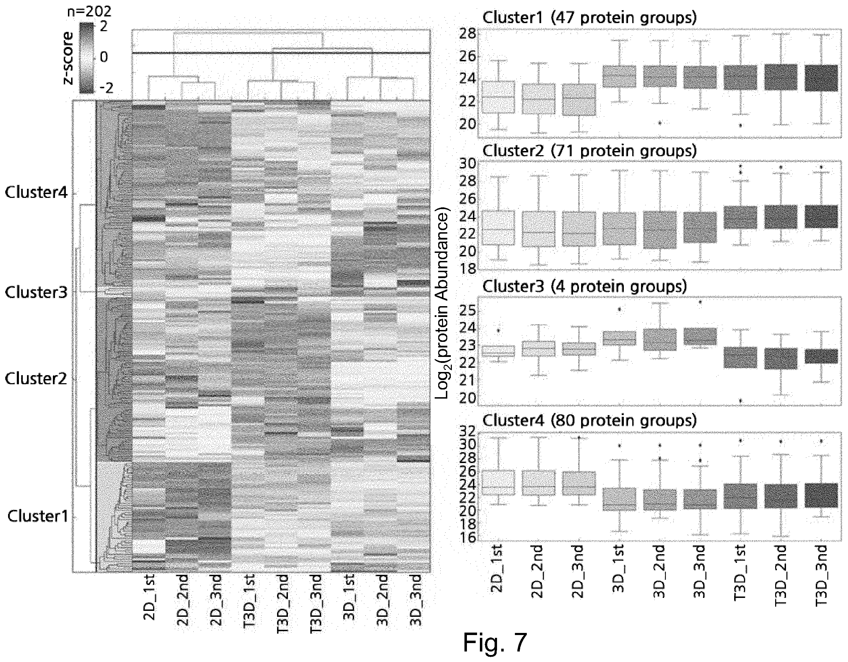

The difference in the protein expression pattern of each sample was quantified by label-free quantitative analysis using the data obtained from the quantitative analysis, and the proteins were divided into four clusters depending on the difference in patterns. From the four clusters, the proteins showing specific difference in the T-3D-EV sample, i.e., the exosomes obtained by the method of the present disclosure, are shown in FIG. 7.

### Comparative analysis of biological characteristics of proteins

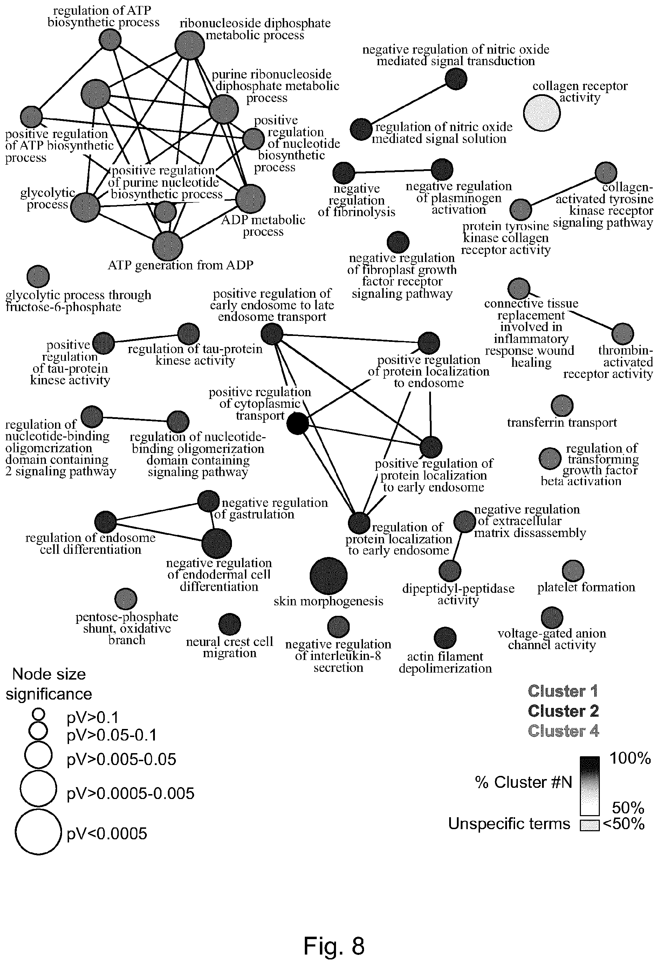

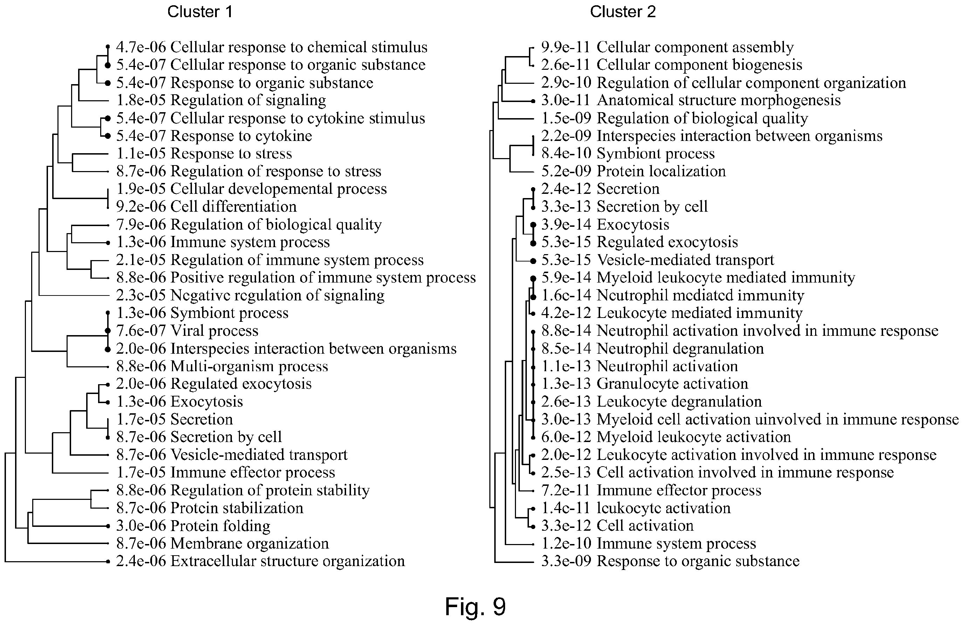

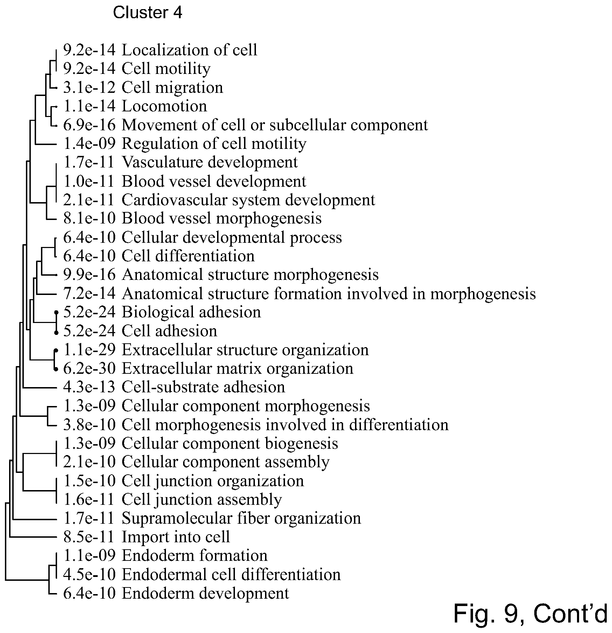

For screening of proteins having specific characteristics in T-3D-EV, the protein expression profile was compared with those of a 3D shaking culture group (3D-EV) and a 2D culture group (2D-EV) without TGF-β3 treatment. As shown in FIG. 8, the proteins showing specific characteristics in T-3D-EV showed the characteristics in glycolytic process, connective tissue replacement involved in inflammatory response wound healing, platelet formation, pentose phosphate pathway, oxidative branch, etc., and showed distinct difference from other control groups. As shown in FIG. 9, immune-associated characteristics were also observed in T-3D.

### Principal component analysis

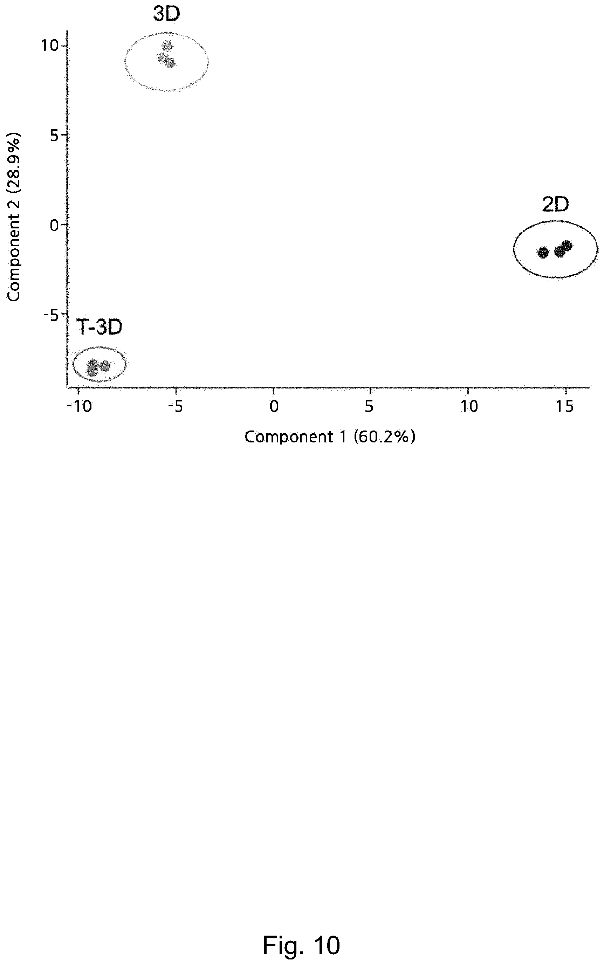

In principal component analysis, the degree of discrimination between sample groups is identified by discrimination indices. The data shown in FIG. 10 were obtained by repeating mass spectroscopy 3 times with different sample treatment. The contribution of the first principal component was 60.2%, and the contribution of the second principal component was 28.9%. As a result, significant difference in patterns was identified in the qualitative and quantitative characteristics of proteins depending on the sample treatment method. Because the distance between 3D-EV and T-3D-EV is smaller than those from 2D-EV, it is thought that the difference in proteins arises from the difference in the 2D method and the 3D method.

### Gene set enrichment analysis

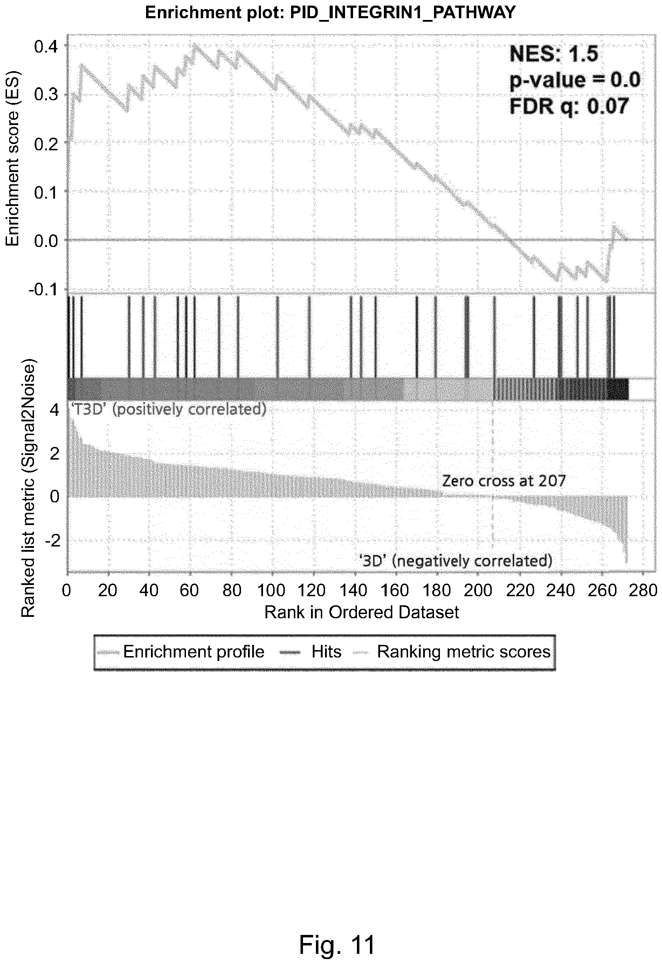

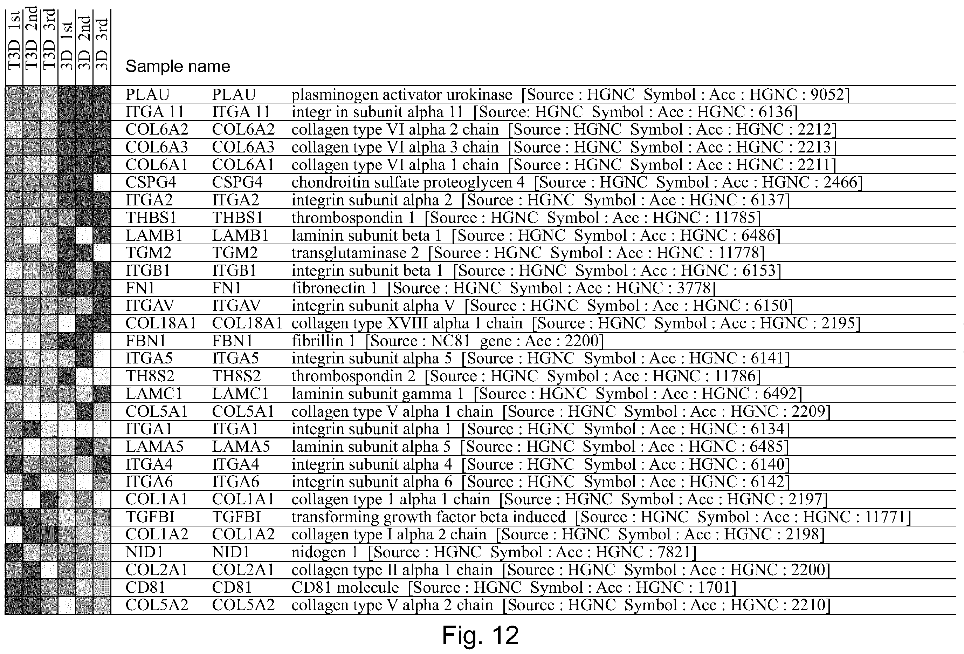

Gene set enrichment analysis was conducted using the GSEA module. The enrichment score allocates a pre-specified class when a gene included in the corresponding set is expressed discriminately according to the phenotype. The normalized enrichment score was calculated by normalizing the enrichment score to the number of genes in the gene set. The nominal p-value is re-calculating the normalized enrichment score in order to replace susceptibility and resistance indices and generate a null distribution. FIG. 11 shows a result of the gene set enrichment analysis and shows the enriched gene sets, normalized enrichment scores (NES) and p-values. The chance that detection is significant is higher as the NES is higher and the p-value is lower. The gene groups enriched in the analysis are shown in FIG. 12.

### Identification of T-3D-EV-specific proteins

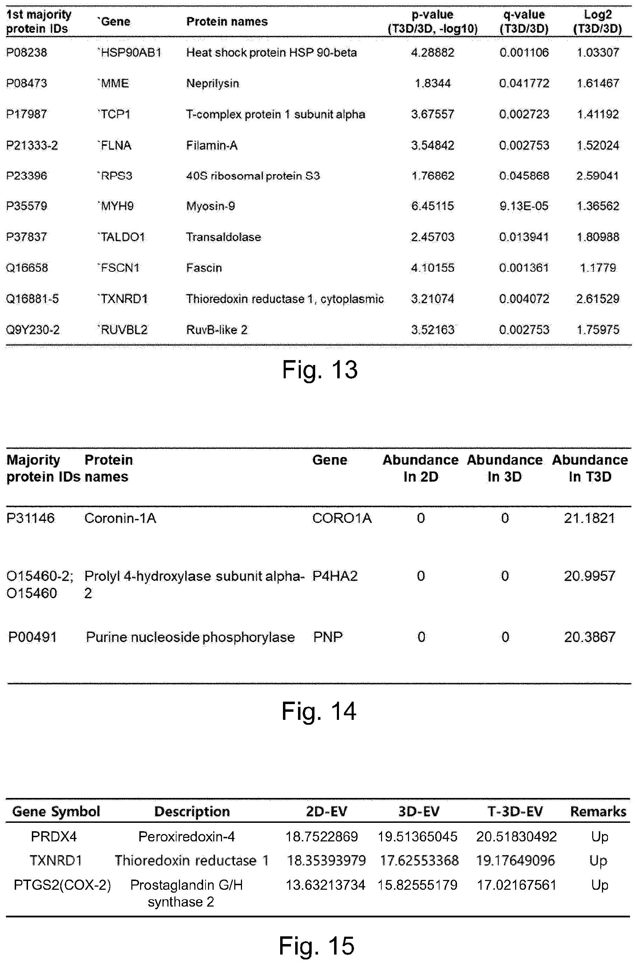

In order to identify the proteins which show significant difference in the group to which TGF-β was added, comparison was performed for sample pairs. The proteins which showed 2 times or more increase or decrease in 3D-EV as compared to T-3D-EV and had Benjamini-Hochberg FDR of 0.05 or lower were screened. In addition, the proteins detected only in the T-3D sample were included. The screened proteins are shown in FIG. 13 and FIG. 14.

As a result of the analysis of proteins existing in EV shown in FIG. 15, antioxidant proteins and inflammation-related factors were expressed.

The representative cellular stress responses are ROS generation and activation of the cellular redox regulatory network thereby. ROS are free radicals which induce the change in the structure and activity of proteins and DNAs by attacking them and, thereby, are involved in DNA repair, cell cycle regulation and cellular growth/apoptosis.

Six types of peroxiredoxins are present in mammals. They are widely distributed in various cells and tissues and function as important antioxidant proteins that remove H₂O₂ generated in tissues. The thioredoxin system is a representative cellular defense system which resolves stresses induced by ROS, etc. It acts as an ROS scavenger of recovering proteins oxidized by ROS through reversible redox reactions and is involved in various cellular defense systems. It is known as a key protein in charge of the cellular defense system acting not only as a survival-inducing factor of cells but also as an anti-apoptotic, anti-inflammatory and immuno-regulatory factor. It was confirmed that the antioxidant proteins peroxiredoxin-4 and thioredoxin reductase are highly expressed in T-3D-EV, i.e., the exosomes obtained by the method of the present disclosure (FIG. 15).

In addition, it was found out that COX-2 is also highly expressed in T-3D-EV (FIG. 15). In general, the inflammatory response is part of the defensive response of body tissues to physical or chemical stimulation or bacterial infection from outside. It is a mechanism for repairing or regenerating damaged tissues. When inflammatory response occurs in the body, inflammatory cells such as macrophages secrete inflammatory mediators such as nitrogen oxide (NO), prostaglandin E2 (PGE2), tumor necrosis factor-α (TNF-α), interleukin-1β (IL-1β), etc. The synthesis of PGE2 begins with the generation of arachidonic acid from the membrane phospholipid by phospholipase A2. Arachidonic acid is converted to prostaglandin G2 by enzymatic action and then to the unstable metabolite prostaglandin H2. These two processes are facilitated by cyclooxygenase (COX). COX exists as two or more isoenzymes. Among them, COX-1 is expressed consistently and is involved in physiological functions such as platelet aggregation, protection of the gastric mucous membrane, regulation of renal function, etc. COX-2 is expressed in response to stimulation such as inflammation and it is known that prostaglandin generated by COX-2 is involved in inflammatory response and cellular proliferation. Therefore, it is though that the high expression of COX-2 and T-3D-EV is because the exosomes of the present disclosure are involved in the regulation of inflammatory response.

While specific exemplary embodiments of the present disclosure have been described in detail, it will be obvious to those having ordinary knowledge in the art that they are mere specific exemplary embodiments and the scope of the present disclosure is not limited by them. It is to be understood that the substantial scope of the present disclosure is defined by the appended claims and their equivalents.

## Claims

1. A method for producing stem cell-derived extracellular vesicles, comprising:
(a) a step of forming cell aggregates by culturing stem cells isolated from a subject; and
(b) a step of three-dimensionally culturing the cell aggregates in a culture medium comprising TGF-β (transforming growth factor beta).

2. The method according to claim 1, wherein the stem cells are mesenchymal stem cells.

3. The method according to claim 1, wherein the step (a) is performed by suspension-culturing the stem cells in a multi-well culture plate.

4. The method according to claim 3, wherein the multi-well culture plate is a microwell plate having wells with a size of 300-500 µm.

5. The method according to claim 3, wherein the suspension culture is performed by seeding 300-500 cells per each well of the multi-well culture plate.

6. The method according to claim 1, wherein the TGF-β is TGF-β3.

7. The method according to claim 1, wherein the step (b) is performed by orbital shaking-culturing the cell aggregate in floating state.

8. The method according to claim 7, wherein the orbital shaking culture is performed at 50-70 rpm.

9. The method according to claim 1, which further comprises a step of isolating the extracellular vesicles from the culture medium obtained in the step (b) through multiple times of centrifugation.

10. The method according to claim 1, wherein the extracellular vesicles have an average diameter of 30-150 nm.

11. Stem cell-derived extracellular vesicles produced by the method according to any of claims 1 to 10.

12. A composition for preventing or treating an inflammatory or autoimmune disease, comprising the stem cell-derived extracellular vesicles according to claim 11 as active ingredients.

13. The composition according to claim 12, wherein the inflammatory or autoimmune disease is rheumatoid arthritis, reactive arthritis, type 1 diabetes, type 2 diabetes, systemic lupus erythematosus, multiple sclerosis, cryptogenic fibrosing alveolitis, polymyositis, dermatomyositis, localized scleroderma, systemic scleroderma, colitis, inflammatory bowel disease, Sjorgen's syndrome, Raynaud's phenomenon, Bechet's disease, Kawasaki's disease, primary biliary sclerosis, primary sclerosing cholangitis, ulcerative colitis, graft-versus-host disease (GVHD) or Crohn's disease.

14. Stem cell-derived extracellular vesicles highly expressing one or more protein selected from a group consisting of peroxiredoxin-4, thioredoxin reductase 1 and prostaglandin G/H synthase 2.

15. The stem cell-derived extracellular vesicles according to claim 14, wherein the extracellular vesicles further highly express one or more protein selected from a group consisting of heat shock protein 90-β (HSP90-β), neprilysin, T-complex protein 1 (TCP1) subunit α, filamin A, 40S ribosomal protein S3, myosin-9, transaldolase, fascin, thioredoxin reductase 1 and RuvB-like 2 (RUVBL 2).

16. The stem cell-derived extracellular vesicles according to claim 14, wherein the extracellular vesicles are positive for one or more protein selected from a group consisting of coronin 1A, prolyl 4-hydroxylase subunit α-2 and purine nucleoside phosphorylase.
